# DEMANDE DE BREVET EUROPEEN

(11) **EP 2 166 095 A2**
(43) Date de publication de la demande: **24.03.2010**
(21) Numéro de dépôt: 09164013.6
(22) Date de dépôt: 14.06.2002
(51) Int. Cl.: C12N 15/113, A61K 31/7088, C07H 21/04, A61P 9/10, A61P 17/00, A61P 35/00

(54) **Oligonucléotides antisens capables d'inhiber la formation des tubes capillaires par les cellules endothéliales**

(30) Priorité: 14.06.2001 FR 0107805
(62) Demande divisionnaire de: 02751246.6
(71) Demandeur: GENE SIGNAL INTERNATIONAL SA, 1066 Epalinges, VD (CH)
(72) Inventeur: Al Mahmood, Salman, 75014 Paris (FR)
(74) Mandataire: de Mareüil-Villette, Caroline

(57) **Abrégé**

La présente invention a pour objet des compositions pharmaceutiques pour inhiber la formation de tubes capillaires par les cellules endothéliales, comprenant au moins un oligonucléotide capable d'inhiber l'expression de la protéine IRS-1. Les oligonucléotides selon l'invention sont indiqués comme agents anti-angiogéniques. Les compositions pharmaceutiques de l'invention sont particulièrement utiles pour le traitement de pathologies liées à l'angiogénèse.

LISTAGE DE SEQUENCES

<110> AL MAHMOOD, SALMAN
<120> Oligonucleotides antisens capables d'inhiber la formation de tubes
capillaires par les cellules endothéliales
<130> B6531-PCT-Juin 2002
<140> PCT/FR02/xxxxx
<141> 2002-06-14
<150> FR01/07805
<151> 2001-06-14
<160> 29
<170> PatentIn version 3.1
<210> 1
<211> 48
<212> DNA
<213> Séquence artificielle
<220>
<221> misc_feature
<222> (1)..(48)
<223> oligonucleotide sens

<400> 1
tcgatgtgac gctactgatg agtccgtgag gacgaaactc tggcctag 48

<210> 2
<211> 35
<212> DNA
<213> Séquence artificielle
<220>
<221> misc_feature
<222> (1)..(35)
<223> Oligonucleotide anti-sens.

<400> 2
tatccggagg gctcgccatg ctgctgcgga gcaga 35

<210> 3
<211> 25
<212> DNA
<213> Séquence artificielle
<220>
<221> misc_feature
<222> (1)..(25)
<223> Oligonucleotide anti-sens.

<400> 3
tatccggagg gctcgccatg ctgct 25

<210> 4
<211> 23
<212> DNA
<213> Séquence artificielle
<220>
<221> misc_feature

<222>  (1)..(23)
<223>  Oligonucleotide anti-sens.


<400>  4
tcgccatgct gctgcggagc aga                                              23



<210>  5
<211>  25
<212>  DNA
<213>  Séquence artificielle
<220>
<221>  misc_feature
<222>  (1)..(25)
<223>  Oligonucleotide anti-sens.


<400>  5
tatccggagg gcctgccatg ctgct                                            25



<210>  6
<211>  24
<212>  DNA
<213>  Séquence artificielle
<220>
<221>  misc_feature
<222>  (1)..(24)
<223>  Oligonucleotide anti-sens.


<400>  6
tatccggagg gcctgccatg ctgc                                             24



<210>  7
<211>  23
<212>  DNA
<213>  Séquence artificielle
<220>
<221>  misc_feature
<222>  (1)..(23)
<223>  Oligonucleotide anti-sens.


<400>  7
tatccggagg gcctgccatg ctg                                              23



<210>  8
<211>  22
<212>  DNA
<213>  Séquence artificielle
<220>
<221>  misc_feature
<222>  (1)..(22)
<223>  Oligonucleotide anti-sens.

<400>  8
tatccggagg gcctgccatg ct                                                22

<210>  9
<211>  21
<212>  DNA
<213>  Séquence artificielle
<220>
<221>  misc_feature
<222>  (1)..(21)
<223>  Oligonucleotide anti-sens.

<400>  9
tatccggagg gcctgccatg c                                                 21

<210>  10
<211>  20
<212>  DNA
<213>  Séquence artificielle
<220>
<221>  misc_feature
<222>  (1)..(20)
<223>  Oligonucleotide anti-sens.

<400>  10
tatccggagg gcctgccatg                                                   20

<210>  11
<211>  19
<212>  DNA
<213>  Séquence artificielle
<220>
<221>  misc_feature
<222>  (1)..(19)
<223>  Oligonucleotide anti-sens.

<400>  11
tatccggagg gcctgccat                                                    19

<210>  12
<211>  18
<212>  DNA
<213>  Séquence artificielle
<220>
<221>  misc_feature
<222>  (1)..(18)
<223>  Oligonucleotide anti-sens.

<400>  12
tatccggagg gcctgcca                                                     18

<210> 13
<211> 17
<212> DNA
<213> Séquence artificielle
<220>
<221> misc_feature
<222> (1)..(17)
<223> Oligonucleotide anti-sens.

<400> 13
tatccggagg gcctgcc 17

<210> 14
<211> 16
<212> DNA
<213> Séquence artificielle
<220>
<221> misc_feature
<222> (1)..(16)
<223> Oligonucleotide anti-sens.

<400> 14
tatccggagg gcctgc 16

<210> 15
<211> 15
<212> DNA
<213> Séquence artificielle
<220>
<221> misc_feature
<222> (1)..(15)
<223> Oligonucleotide anti-sens.

<400> 15
tatccggagg gcctg 15

<210> 16
<211> 14
<212> DNA
<213> Séquence artificielle
<220>
<221> misc_feature
<222> (1)..(14)
<223> Oligonucleotide anti-sens.

<400> 16
tatccggagg gcct 14

<210>  17
<211>  13
<212>  DNA
<213>  Séquence artificielle
<220>
<221>  misc_feature
<222>  (1)..(13)
<223>  Oligonucleotide anti-sens.

<400>  17
tatccggagg gcc                                                          13

<210>  18
<211>  12
<212>  DNA
<213>  Séquence artificielle
<220>
<221>  misc_feature
<222>  (1)..(12)
<223>  Ologonucleotide anti-sens.

<400>  18
tatccggagg gc                                                           12

<210>  19
<211>  22
<212>  DNA
<213>  Séquence artificielle
<220>
<221>  misc_feature
<222>  (1)..(22)
<223>  Oligonucleotide anti-sens.

<400>  19
ccggagggcc tgccatgctg ct                                                22

<210>  20
<211>  19
<212>  DNA
<213>  Séquence artificielle
<220>
<221>  misc_feature
<222>  (1)..(19)
<223>  Oigonucleotide anti-sens.

<400>  20
gagggcctgc catgctgct                                                    19

<210>  21
<211>  16
<212>  DNA

<213>  Séquence artificielle
<220>
<221>  misc_feature
<222>  (1)..(16)
<223>  Oligonucleotide anti-sens.

<400>  21
ggcctgccat gctgct                                                    16

<210>  22
<211>  13
<212>  DNA
<213>  Séquence artificielle
<220>
<221>  misc_feature
<222>  (1)..(13)
<223>  Oligonucleotide anti-sens.

<400>  22
ctgccatgct gct                                                       13

<210>  23
<211>  12
<212>  DNA
<213>  Séquence artificielle
<220>
<221>  misc_feature
<222>  (1)..(12)
<223>  Oligonucleotide anti-sens.

<400>  23
tgccatgctg ct                                                        12

<210>  24
<211>  14
<212>  DNA
<213>  Séquence artificielle
<220>
<221>  misc_feature
<222>  (1)..(14)
<223>  Oligonucleotide anti-sens;

<400>  24
tttttttttt ttgg                                                      14

<210>  25
<211>  14
<212>  DNA
<213>  Séquence artificielle.
<220>
<221>  misc_feature

<222>  (1)..(14)
<223>  Oligonucleotide anti-sens.


<400>  25
tttttttttt ttag                                                          14



<210>  26
<211>  14
<212>  DNA
<213>  Séquence artificielle
<220>
<221>  misc_feature
<222>  (1)..(14)
<223>  Oligonucleotide anti-sens.


<400>  26
tttttttttt ttcg                                                          14



<210>  27
<211>  326
<212>  DNA
<213>  Homo sapiens
<220>
<221>  misc_feature
<222>  (1)..(326)
<223>  Fragment du gène IRS-1 codant pour le substrat du récepteur de l'
       insuline.


<400>  27
gtgccgagct gagttcctta taagaattaa tcttaatttt gtattttttc ctgtaagaca      60

ataggccatg ttaattaaac tgaagaagga tatatttggc tgggtgtttt caaatgtcag     120

cttaaaattg gtaattgaat ggaagcaaaa ttataagaag aggaaattaa agtcttccat     180

tgcatgtatt gtaaacagaa ggagatgggt gattccttca attcaaaagc tctctttgga     240

atgaacaatg tgggcgtttg taaattctgg aaatgtcttt ctattcataa taaactagat     300

actgttgatc ttttaaaaaa aaaaaa                                          326



<210>  28
<211>  5800
<212>  DNA
<213>  Homo sapiens
<220>
<221>  CDS
<222>  (1022)..(4750)
<223>


<400>  28
cggcggcgcg gtcggagggg gccggcgcgc agagccagac gccgccgctt gttttggttg      60

gggctctcgg caactctccg aggaggagga ggaggaggga ggaggggaga agtaactgca 120

gcggcagcgc ctcccgagga acaggcgtct tccccgaacc cttcccaaac ctcccccatc 180

ccctctcgcc cttgtcccct cccctcctcc ccagccgcct ggagcgaggg gcagggatga 240

gtctgtccct ccggccggtc cccagctgca gtggctgccc ggtatcgttt cgcatggaaa 300

agccactttc tccacccgcc gagatgggcc cggatggggg ctgcagagga cgcgcccgcg 360

ggcggcggca gcagcagcag cagcagcagc agcaacagca acagccgcag cgccgcggtc 420

tctgcgactg agctggtatt tgggcggctg gtggcggctg ggacggttgg ggggtgggag 480

gaggcgaagg aggagggaga accccgtgca acgttgggac ttggcaaccc gcctccccct 540

gcccaaggat atttaatttg cctcgggaat cgctgcttcc agaggggaac tcaggaggga 600

aggcgcgcgc gcgcgcgcgc tcctggaggg gcaccgcagg gacccccgac tgtcgcctcc 660

ctgtgccgga ctccagccgg ggcgacgaga gatgcatctt cgctccttcc tggtggcggc 720

ggcggctgag aggagacttg gctctcggag gatcggggct gccctcaccc cggacgcact 780

gcctccccgc cgggcgtgaa gcgcccgaaa actccggtcg ggctctctcc tgggctcagc 840

agctgcgtcc tccttcagct gcccctcccc ggcgcggggg gcggcgtgga tttcagagtc 900

ggggtttctg ctgcctccag ccctgtttgc atgtgccggg ccgcggcgag gagcctccgc 960

cccccacccg gttgtttttc ggagcctccc tctgctcagc gttggtggtg gcggtggcag 1020

c atg gcg agc cct ccg gag agc gat ggc ttc tcg gac gtg cgc aag gtg 1069
Met Ala Ser Pro Pro Glu Ser Asp Gly Phe Ser Asp Val Arg Lys Val
1               5                   10                  15

ggc tac ctg cgc aaa ccc aag agc atg cac aaa cgc ttc ttc gta ctg 1117
Gly Tyr Leu Arg Lys Pro Lys Ser Met His Lys Arg Phe Phe Val Leu
            20                  25                  30

cgc gcg gcc agc gag gct ggg ggc ccg gcg cgc ctc gag tac tac gag 1165
Arg Ala Ala Ser Glu Ala Gly Gly Pro Ala Arg Leu Glu Tyr Tyr Glu
        35                  40                  45

aac gag aag aag tgg cgg cac aag tcg agc gcc ccc aaa cgc tcg atc 1213
Asn Glu Lys Lys Trp Arg His Lys Ser Ser Ala Pro Lys Arg Ser Ile
    50                  55                  60

ccc ctt gag agc tgc ttc aac atc aac aag cgg gct gac tcc aag aac 1261
Pro Leu Glu Ser Cys Phe Asn Ile Asn Lys Arg Ala Asp Ser Lys Asn
65                  70                  75                  80

aag cac ctg gtg gct ctc tac acc cgg gac gag cac ttt gcc atc gcg 1309
Lys His Leu Val Ala Leu Tyr Thr Arg Asp Glu His Phe Ala Ile Ala
                85                  90                  95

gcg gac agc gag gcc gag caa gac agc tgg tac cag gct ctc cta cag 1357
Ala Asp Ser Glu Ala Glu Gln Asp Ser Trp Tyr Gln Ala Leu Leu Gln
            100                 105                 110

ctg cac aac cgt gct aag ggc cac cac gac gga gct gcg gcc ctc ggg 1405
Leu His Asn Arg Ala Lys Gly His His Asp Gly Ala Ala Ala Leu Gly
        115                 120                 125

gcg gga ggt ggt ggg ggc agc tgc agc ggc agc tcc ggc ctt ggt gag 1453
Ala Gly Gly Gly Gly Gly Ser Cys Ser Gly Ser Ser Gly Leu Gly Glu
    130                 135                 140

gct ggg gag gac ttg agc tac ggt gac gtg ccc cca gga ccc gca ttc 1501
Ala Gly Glu Asp Leu Ser Tyr Gly Asp Val Pro Pro Gly Pro Ala Phe
145                 150                 155                 160

aaa gag gtc tgg caa gtg atc ctg aag ccc aag ggc ctg ggt cag aca 1549
Lys Glu Val Trp Gln Val Ile Leu Lys Pro Lys Gly Leu Gly Gln Thr
                165                 170                 175

aag aac ctg att ggt atc tac cgc ctt tgc ctg acc agc aag acc atc 1597
Lys Asn Leu Ile Gly Ile Tyr Arg Leu Cys Leu Thr Ser Lys Thr Ile
            180                 185                 190

agc ttc gtg aag ctg aac tcg gag gca gcg gcc gtg gtg ctg cag ctg 1645
Ser Phe Val Lys Leu Asn Ser Glu Ala Ala Ala Val Val Leu Gln Leu
        195                 200                 205

atg aac atc agg cgc tgt ggc cac tcg gaa aac ttc ttc ttc atc gag 1693
Met Asn Ile Arg Arg Cys Gly His Ser Glu Asn Phe Phe Phe Ile Glu
    210                 215                 220

gtg ggc cgt tct gcc gtg acg ggg ccc ggg gag ttc tgg atg cag gtg 1741
Val Gly Arg Ser Ala Val Thr Gly Pro Gly Glu Phe Trp Met Gln Val
225                 230                 235                 240

gat gac tct gtg gtg gcc cag aac atg cac gag acc atc ctg gag gcc 1789
Asp Asp Ser Val Val Ala Gln Asn Met His Glu Thr Ile Leu Glu Ala
                245                 250                 255

atg cgg gcc atg agt gat gag ttc cgc cct cgc agc aag agc cag tcc 1837
Met Arg Ala Met Ser Asp Glu Phe Arg Pro Arg Ser Lys Ser Gln Ser
            260                 265                 270

tcg tcc aac tgc tct aac ccc atc agc gtc ccc ctg cgc cgg cac cat 1885
Ser Ser Asn Cys Ser Asn Pro Ile Ser Val Pro Leu Arg Arg His His
        275                 280                 285

ctc aac aat ccc ccg ccc agc cag gtg ggg ctg acc cgc cga tca cgc 1933
Leu Asn Asn Pro Pro Pro Ser Gln Val Gly Leu Thr Arg Arg Ser Arg
    290                 295                 300

act gag agc atc acc gcc acc tcc ccg gcc agc atg gtg ggc ggg aag 1981
Thr Glu Ser Ile Thr Ala Thr Ser Pro Ala Ser Met Val Gly Gly Lys
305                 310                 315                 320

cca ggc tcc ttc cgt gtc cgc gcc tcc agt gac ggc gaa ggc acc atg 2029
Pro Gly Ser Phe Arg Val Arg Ala Ser Ser Asp Gly Glu Gly Thr Met
                325                 330                 335

tcc cgc cca gcc tcg gtg gac ggc agc cct gtg agt ccc agc acc aac 2077
Ser Arg Pro Ala Ser Val Asp Gly Ser Pro Val Ser Pro Ser Thr Asn
            340                 345                 350

aga acc cac gcc cac cgg cat cgg ggc agc gcc cgg ctg cac ccc ccg 2125
Arg Thr His Ala His Arg His Arg Gly Ser Ala Arg Leu His Pro Pro
        355                 360                 365

ctc aac cac agc cgc tcc atc ccc atg ccg gct tcc cgc tgc tcg cct 2173
Leu Asn His Ser Arg Ser Ile Pro Met Pro Ala Ser Arg Cys Ser Pro

370                     375                     380

tcg gcc acc agc ccg gtc agt ctg tcg tcc agt agc acc agt ggc cat     2221
Ser Ala Thr Ser Pro Val Ser Leu Ser Ser Ser Ser Thr Ser Gly His
385                 390                 395                 400

ggc tcc acc tcg gat tgt ctc ttc cca cgg cga tct agt gct tcg gtg     2269
Gly Ser Thr Ser Asp Cys Leu Phe Pro Arg Arg Ser Ser Ala Ser Val
                405                 410                 415

tct ggt tcc ccc agc gat ggc ggt ttc atc tcc tcg gat gag tat ggc     2317
Ser Gly Ser Pro Ser Asp Gly Gly Phe Ile Ser Ser Asp Glu Tyr Gly
            420                 425                 430

tcc agt ccc tgc gat ttc cgg agt tcc ttc cgc agt gtc act ccg gat     2365
Ser Ser Pro Cys Asp Phe Arg Ser Ser Phe Arg Ser Val Thr Pro Asp
        435                 440                 445

tcc ctg ggc cac acc cca cca gcc cgc ggt gag gag gag cta agc aac     2413
Ser Leu Gly His Thr Pro Pro Ala Arg Gly Glu Glu Glu Leu Ser Asn
    450                 455                 460

tat atc tgc atg ggt ggc aag ggg ccc tcc acc ctg acc gcc ccc aac     2461
Tyr Ile Cys Met Gly Gly Lys Gly Pro Ser Thr Leu Thr Ala Pro Asn
465                 470                 475                 480

ggt cac tac att ttg tct cgg ggt ggc aat ggc cac cgc tgc acc cca     2509
Gly His Tyr Ile Leu Ser Arg Gly Gly Asn Gly His Arg Cys Thr Pro
                485                 490                 495

gga aca ggc ttg ggc acg agt cca gcc ttg gct ggg gat gaa gca gcc     2557
Gly Thr Gly Leu Gly Thr Ser Pro Ala Leu Ala Gly Asp Glu Ala Ala
            500                 505                 510

agt gct gca gat ctg gat aat cgg ttc cga aag aga act cac tcg gca     2605
Ser Ala Ala Asp Leu Asp Asn Arg Phe Arg Lys Arg Thr His Ser Ala
        515                 520                 525

ggc aca tcc cct acc att acc cac cag aag acc ccg tcc cag tcc tca     2653
Gly Thr Ser Pro Thr Ile Thr His Gln Lys Thr Pro Ser Gln Ser Ser
    530                 535                 540

gtg gct tcc att gag gag tac aca gag atg atg cct gcc tac cca cca     2701
Val Ala Ser Ile Glu Glu Tyr Thr Glu Met Met Pro Ala Tyr Pro Pro
545                 550                 555                 560

gga ggt ggc agt gga ggc cga ctg ccg gga cac agg cac tcc gcc ttc     2749
Gly Gly Gly Ser Gly Gly Arg Leu Pro Gly His Arg His Ser Ala Phe
                565                 570                 575

gtg ccc acc cgc tcc tac cca gag gag ggt ctg gaa atg cac ccc ttg     2797
Val Pro Thr Arg Ser Tyr Pro Glu Glu Gly Leu Glu Met His Pro Leu
            580                 585                 590

gag cgt cgg ggg ggg cac cac cgc cca gac agc tcc acc ctc cac acg     2845
Glu Arg Arg Gly Gly His His Arg Pro Asp Ser Ser Thr Leu His Thr
        595                 600                 605

gat gat ggc tac atg ccc atg tcc cca ggg gtg gcc cca gtg ccc agt     2893
Asp Asp Gly Tyr Met Pro Met Ser Pro Gly Val Ala Pro Val Pro Ser
    610                 615                 620

ggc cga aag ggc agt gga gac tat atg ccc atg agc ccc aag agc gta     2941

Gly Arg Lys Gly Ser Gly Asp Tyr Met Pro Met Ser Pro Lys Ser Val
625                 630                 635                 640

tct gcc cca cag cag atc atc aat ccc atc aga cgc cat ccc cag aga      2989
Ser Ala Pro Gln Gln Ile Ile Asn Pro Ile Arg Arg His Pro Gln Arg
                645                 650                 655

gtg gac ccc aat ggc tac atg atg atg tcc ccc agc ggt ggc tgc tct      3037
Val Asp Pro Asn Gly Tyr Met Met Met Ser Pro Ser Gly Gly Cys Ser
            660                 665                 670

cct gac att gga ggt ggc ccc agc agc agc agc agc agc agc aac gcc      3085
Pro Asp Ile Gly Gly Gly Pro Ser Ser Ser Ser Ser Ser Ser Asn Ala
        675                 680                 685

gtc cct tcc ggg acc agc tat gga aag ctg tgg aca aac ggg gta ggg      3133
Val Pro Ser Gly Thr Ser Tyr Gly Lys Leu Trp Thr Asn Gly Val Gly
    690                 695                 700

ggc cac cac tct cat gtc ttg cct cac ccc aaa ccc cca gtg gag agc      3181
Gly His His Ser His Val Leu Pro His Pro Lys Pro Pro Val Glu Ser
705                 710                 715                 720

agc ggt ggt aag ctc tta cct tgc aca ggt gac tac atg aac atg tca      3229
Ser Gly Gly Lys Leu Leu Pro Cys Thr Gly Asp Tyr Met Asn Met Ser
                725                 730                 735

cca gtg ggg gac tcc aac acc agc agc ccc tcc gac tgc tac tac ggc      3277
Pro Val Gly Asp Ser Asn Thr Ser Ser Pro Ser Asp Cys Tyr Tyr Gly
            740                 745                 750

cct gag gac ccc cag cac aag cca gtc ctc tcc tac tac tca ttg cca      3325
Pro Glu Asp Pro Gln His Lys Pro Val Leu Ser Tyr Tyr Ser Leu Pro
        755                 760                 765

aga tcc ttt aag cac acc cag cgc ccc ggg gag ccg gag gag ggt gcc      3373
Arg Ser Phe Lys His Thr Gln Arg Pro Gly Glu Pro Glu Glu Gly Ala
    770                 775                 780

cgg cat cag cac ctc cgc ctt tcc act agc tct ggt cgc ctt ctc tat      3421
Arg His Gln His Leu Arg Leu Ser Thr Ser Ser Gly Arg Leu Leu Tyr
785                 790                 795                 800

gct gca aca gca gat gat tct tcc tct tcc acc agc agc gac agc ctg      3469
Ala Ala Thr Ala Asp Asp Ser Ser Ser Ser Thr Ser Ser Asp Ser Leu
                805                 810                 815

ggt ggg gga tac tgc ggg gct agg ctg gag ccc agc ctt cca cat ccc      3517
Gly Gly Gly Tyr Cys Gly Ala Arg Leu Glu Pro Ser Leu Pro His Pro
            820                 825                 830

cac cat cag gtt ctg cag ccc cat ctg cct cga aag gtg gac aca gct      3565
His His Gln Val Leu Gln Pro His Leu Pro Arg Lys Val Asp Thr Ala
        835                 840                 845

gct cag acc aat agc cgc ctg gcc cgg ccc acg agg ctg tcc ctg ggg      3613
Ala Gln Thr Asn Ser Arg Leu Ala Arg Pro Thr Arg Leu Ser Leu Gly
    850                 855                 860

gat ccc aag gcc agc acc tta cct cgg gcc cga gag cag cag cag cag      3661
Asp Pro Lys Ala Ser Thr Leu Pro Arg Ala Arg Glu Gln Gln Gln Gln
865                 870                 875                 880

cag cag ccc ttg ctg cac cct cca gag ccc aag agc ccg ggg gaa tat 3709
Gln Gln Pro Leu Leu His Pro Pro Glu Pro Lys Ser Pro Gly Glu Tyr
                885                 890                 895

gtc aat att gaa ttt ggg agt gat cag tct ggc tac ttg tct ggc ccg 3757
Val Asn Ile Glu Phe Gly Ser Asp Gln Ser Gly Tyr Leu Ser Gly Pro
            900                 905                 910

gtg gct ttc cac agc tca cct tct gtc agg tgt cca tcc cag ctc cag 3805
Val Ala Phe His Ser Ser Pro Ser Val Arg Cys Pro Ser Gln Leu Gln
        915                 920                 925

cca gct ccc aga gag gaa gag act ggc act gag gag tac atg aag atg 3853
Pro Ala Pro Arg Glu Glu Glu Thr Gly Thr Glu Glu Tyr Met Lys Met
    930                 935                 940

gac ctg ggg ccg ggc cgg agg gca gcc tgg cag gag agc act ggg gtc 3901
Asp Leu Gly Pro Gly Arg Arg Ala Ala Trp Gln Glu Ser Thr Gly Val
945                 950                 955                 960

gag atg ggc aga ctg ggc cct gca cct ccc ggg gct gct agc att tgc 3949
Glu Met Gly Arg Leu Gly Pro Ala Pro Pro Gly Ala Ala Ser Ile Cys
                965                 970                 975

agg cct acc cgg gca gtg ccc agc agc cgg ggt gac tac atg acc atg 3997
Arg Pro Thr Arg Ala Val Pro Ser Ser Arg Gly Asp Tyr Met Thr Met
            980                 985                 990

cag atg agt tgt ccc cgt cag agc tac gtg gac acc tcg cca gct gcc 4045
Gln Met Ser Cys Pro Arg Gln Ser Tyr Val Asp Thr Ser Pro Ala Ala
        995                 1000                1005

cct gta agc tat gct gac atg cga aca ggc att gct gca gag gag 4090
Pro Val Ser Tyr Ala Asp Met Arg Thr Gly Ile Ala Ala Glu Glu
    1010                1015                1020

gtg agc ctg ccc agg gcc acc atg gct gct gcc tcc tca tcc tca 4135
Val Ser Leu Pro Arg Ala Thr Met Ala Ala Ala Ser Ser Ser Ser
    1025                1030                1035

gca gcc tct gct tcc ccg act ggg cct caa ggg gca gca gag ctg 4180
Ala Ala Ser Ala Ser Pro Thr Gly Pro Gln Gly Ala Ala Glu Leu
    1040                1045                1050

gct gcc cac tcg tcc ctg ctg ggg ggc cca caa gga cct ggg ggc 4225
Ala Ala His Ser Ser Leu Leu Gly Gly Pro Gln Gly Pro Gly Gly
    1055                1060                1065

atg agc gcc ttc acc cgg gtg aac ctc agt cct aac cgc aac cag 4270
Met Ser Ala Phe Thr Arg Val Asn Leu Ser Pro Asn Arg Asn Gln
    1070                1075                1080

agt gcc aaa gtg atc cgt gca gac cca caa ggg tgc cgg cgg agg 4315
Ser Ala Lys Val Ile Arg Ala Asp Pro Gln Gly Cys Arg Arg Arg
    1085                1090                1095

cat agc tcc gag act ttc tcc tca aca ccc agt gcc acc cgg gtg 4360
His Ser Ser Glu Thr Phe Ser Ser Thr Pro Ser Ala Thr Arg Val
    1100                1105                1110

ggc aac aca gtg ccc ttt gga gcg ggg gca gca gta ggg ggc ggt 4405
Gly Asn Thr Val Pro Phe Gly Ala Gly Ala Ala Val Gly Gly Gly
    1115                1120                1125

ggc ggt agc agc agc agc agc gag gat gtg aaa cgc cac agc tct 4450
Gly Gly Ser Ser Ser Ser Ser Glu Asp Val Lys Arg His Ser Ser
    1130                1135                1140

gct tcc ttt gag aat gtg tgg ctg agg cct ggg gag ctt ggg gga 4495
Ala Ser Phe Glu Asn Val Trp Leu Arg Pro Gly Glu Leu Gly Gly
    1145                1150                1155

gcc ccc aag gag cca gcc aaa ctg tgt ggg gct gct ggg ggt ttg 4540
Ala Pro Lys Glu Pro Ala Lys Leu Cys Gly Ala Ala Gly Gly Leu
    1160                1165                1170

gag aat ggt ctt aac tac ata gac ctg gat ttg gtc aag gac ttc 4585
Glu Asn Gly Leu Asn Tyr Ile Asp Leu Asp Leu Val Lys Asp Phe
    1175                1180                1185

aaa cag tgc cct cag gag tgc acc cct gaa ccg cag cct ccc cca 4630
Lys Gln Cys Pro Gln Glu Cys Thr Pro Glu Pro Gln Pro Pro Pro
    1190                1195                1200

ccc cca ccc cct cat caa ccc ctg ggc agc ggt gag agc agc tcc 4675
Pro Pro Pro Pro His Gln Pro Leu Gly Ser Gly Glu Ser Ser Ser
    1205                1210                1215

acc cgc cgc tca agt gag gat tta agc gcc tat gcc agc atc agt 4720
Thr Arg Arg Ser Ser Glu Asp Leu Ser Ala Tyr Ala Ser Ile Ser
    1220                1225                1230

ttc cag aag cag cca gag gac cgt cag tag ctcaactgga catcacagca 4770
Phe Gln Lys Gln Pro Glu Asp Arg Gln
    1235                1240

gaatgaagac ctaaatgacc tcagcaaatc ctcttctaac tcatgggtac ccagactcta 4830

aatatttcat gattcacaac taggacctca tatcttcctc atcagtagat ggtacgatgc 4890

atccatttca gtttgtttac tttatccaat cctcaggatt tcattgactg aactgcacgt 4950

tctatattgt gccaagcgaa aaaaaaaaat gcactgtgac accagaataa tgagtctgca 5010

taaacttcat cttcaacctt aaggacttag ctggccacag tgagctgatg tgcccaccac 5070

cgtgtcatga gagaatgggt ttactctcaa tgcattttca agatacattt catctgctgc 5130

tgaaactgtg tacgacaaag catcattgta aattatttca tacaaaactg ttcacgttgg 5190

gtggagagag tattaaatat ttaacatagg ttttgattta tatgtgtaat tttttaaatg 5250

aaaatgtaac ttttcttaca gcacatcttt tttttggatg tgggatggag gtatacaatg 5310

ttctgttgta aagagtggag caaatgctta aaacaaggct taaaagagta gaatagggta 5370

tgatccttgt tttaagattg taattcagaa aacataatat aagaatcata gtgccataga 5430

tggttctcaa ttgtatagtt atatttgctg atactatctc ttgtcatata aacctgatgt 5490

tgagctgagt tccttataag aattaatctt aattttgtat tttttcctgt aagacaatag 5550

gccatgttaa ttaaactgaa gaaggatata tttggctggg tgttttcaaa tgtcagctta 5610

aaattggtaa ttgaatggaa gcaaaattat aagaagagga aattaaagtc ttccattgca 5670

tgtattgtaa acagaaggag atgggtgatt ccttcaattc aaaagctctc tttggaatga 5730

acaatgtggg cgtttgtaaa ttctggaaat gtctttctat tcataataaa ctagatactg 5790

ttgatctttt 5800

<210> 29
<211> 1242
<212> PRT
<213> Homo sapiens
<400> 29

Met Ala Ser Pro Pro Glu Ser Asp Gly Phe Ser Asp Val Arg Lys Val
1               5                   10                  15

Gly Tyr Leu Arg Lys Pro Lys Ser Met His Lys Arg Phe Phe Val Leu
            20                  25                  30

Arg Ala Ala Ser Glu Ala Gly Gly Pro Ala Arg Leu Glu Tyr Tyr Glu
        35                  40                  45

Asn Glu Lys Lys Trp Arg His Lys Ser Ser Ala Pro Lys Arg Ser Ile
    50                  55                  60

Pro Leu Glu Ser Cys Phe Asn Ile Asn Lys Arg Ala Asp Ser Lys Asn
65                  70                  75                  80

Lys His Leu Val Ala Leu Tyr Thr Arg Asp Glu His Phe Ala Ile Ala
                85                  90                  95

Ala Asp Ser Glu Ala Glu Gln Asp Ser Trp Tyr Gln Ala Leu Leu Gln
            100                 105                 110

Leu His Asn Arg Ala Lys Gly His His Asp Gly Ala Ala Ala Leu Gly
        115                 120                 125

Ala Gly Gly Gly Gly Gly Ser Cys Ser Gly Ser Ser Gly Leu Gly Glu
    130                 135                 140

Ala Gly Glu Asp Leu Ser Tyr Gly Asp Val Pro Pro Gly Pro Ala Phe
145                 150                 155                 160

Lys Glu Val Trp Gln Val Ile Leu Lys Pro Lys Gly Leu Gly Gln Thr
                165                 170                 175

Lys Asn Leu Ile Gly Ile Tyr Arg Leu Cys Leu Thr Ser Lys Thr Ile
            180                 185                 190

Ser Phe Val Lys Leu Asn Ser Glu Ala Ala Ala Val Val Leu Gln Leu
        195                 200                 205

Met Asn Ile Arg Arg Cys Gly His Ser Glu Asn Phe Phe Phe Ile Glu
    210                 215                 220

Val Gly Arg Ser Ala Val Thr Gly Pro Gly Glu Phe Trp Met Gln Val
225                 230                 235                 240

Asp Asp Ser Val Val Ala Gln Asn Met His Glu Thr Ile Leu Glu Ala
                245                 250                 255

Met Arg Ala Met Ser Asp Glu Phe Arg Pro Arg Ser Lys Ser Gln Ser

            260                 265                 270

Ser Ser Asn Cys Ser Asn Pro Ile Ser Val Pro Leu Arg Arg His His
        275                 280                 285

Leu Asn Asn Pro Pro Pro Ser Gln Val Gly Leu Thr Arg Arg Ser Arg
    290                 295                 300

Thr Glu Ser Ile Thr Ala Thr Ser Pro Ala Ser Met Val Gly Gly Lys
305                 310                 315                 320

Pro Gly Ser Phe Arg Val Arg Ala Ser Ser Asp Gly Glu Gly Thr Met
                325                 330                 335

Ser Arg Pro Ala Ser Val Asp Gly Ser Pro Val Ser Pro Ser Thr Asn
            340                 345                 350

Arg Thr His Ala His Arg His Arg Gly Ser Ala Arg Leu His Pro Pro
        355                 360                 365

Leu Asn His Ser Arg Ser Ile Pro Met Pro Ala Ser Arg Cys Ser Pro
    370                 375                 380

Ser Ala Thr Ser Pro Val Ser Leu Ser Ser Ser Ser Thr Ser Gly His
385                 390                 395                 400

Gly Ser Thr Ser Asp Cys Leu Phe Pro Arg Arg Ser Ser Ala Ser Val
                405                 410                 415

Ser Gly Ser Pro Ser Asp Gly Gly Phe Ile Ser Ser Asp Glu Tyr Gly
            420                 425                 430

Ser Ser Pro Cys Asp Phe Arg Ser Ser Phe Arg Ser Val Thr Pro Asp
        435                 440                 445

Ser Leu Gly His Thr Pro Pro Ala Arg Gly Glu Glu Glu Leu Ser Asn
    450                 455                 460

Tyr Ile Cys Met Gly Gly Lys Gly Pro Ser Thr Leu Thr Ala Pro Asn
465                 470                 475                 480

Gly His Tyr Ile Leu Ser Arg Gly Gly Asn Gly His Arg Cys Thr Pro
                485                 490                 495

Gly Thr Gly Leu Gly Thr Ser Pro Ala Leu Ala Gly Asp Glu Ala Ala
            500                 505                 510

Ser Ala Ala Asp Leu Asp Asn Arg Phe Arg Lys Arg Thr His Ser Ala
        515                 520                 525

Gly Thr Ser Pro Thr Ile Thr His Gln Lys Thr Pro Ser Gln Ser Ser
    530                 535                 540

Val Ala Ser Ile Glu Glu Tyr Thr Glu Met Met Pro Ala Tyr Pro Pro
545                 550                 555                 560

Gly Gly Gly Ser Gly Gly Arg Leu Pro Gly His Arg His Ser Ala Phe
                565                 570                 575

Val Pro Thr Arg Ser Tyr Pro Glu Glu Gly Leu Glu Met His Pro Leu
            580                 585                 590

Glu Arg Arg Gly Gly His His Arg Pro Asp Ser Ser Thr Leu His Thr

595                 600                 605

Asp Asp Gly Tyr Met Pro Met Ser Pro Gly Val Ala Pro Val Pro Ser
    610                 615                 620

Gly Arg Lys Gly Ser Gly Asp Tyr Met Pro Met Ser Pro Lys Ser Val
625                 630                 635                 640

Ser Ala Pro Gln Gln Ile Ile Asn Pro Ile Arg Arg His Pro Gln Arg
                645                 650                 655

Val Asp Pro Asn Gly Tyr Met Met Met Ser Pro Ser Gly Gly Cys Ser
            660                 665                 670

Pro Asp Ile Gly Gly Gly Pro Ser Ser Ser Ser Ser Ser Ser Asn Ala
        675                 680                 685

Val Pro Ser Gly Thr Ser Tyr Gly Lys Leu Trp Thr Asn Gly Val Gly
    690                 695                 700

Gly His His Ser His Val Leu Pro His Pro Lys Pro Pro Val Glu Ser
705                 710                 715                 720

Ser Gly Gly Lys Leu Leu Pro Cys Thr Gly Asp Tyr Met Asn Met Ser
                725                 730                 735

Pro Val Gly Asp Ser Asn Thr Ser Ser Pro Ser Asp Cys Tyr Tyr Gly
            740                 745                 750

Pro Glu Asp Pro Gln His Lys Pro Val Leu Ser Tyr Tyr Ser Leu Pro
        755                 760                 765

Arg Ser Phe Lys His Thr Gln Arg Pro Gly Glu Pro Glu Glu Gly Ala
    770                 775                 780

Arg His Gln His Leu Arg Leu Ser Thr Ser Ser Gly Arg Leu Leu Tyr
785                 790                 795                 800

Ala Ala Thr Ala Asp Asp Ser Ser Ser Ser Thr Ser Ser Asp Ser Leu
                805                 810                 815

Gly Gly Gly Tyr Cys Gly Ala Arg Leu Glu Pro Ser Leu Pro His Pro
            820                 825                 830

His His Gln Val Leu Gln Pro His Leu Pro Arg Lys Val Asp Thr Ala
        835                 840                 845

Ala Gln Thr Asn Ser Arg Leu Ala Arg Pro Thr Arg Leu Ser Leu Gly
    850                 855                 860

Asp Pro Lys Ala Ser Thr Leu Pro Arg Ala Arg Glu Gln Gln Gln Gln
865                 870                 875                 880

Gln Gln Pro Leu Leu His Pro Pro Glu Pro Lys Ser Pro Gly Glu Tyr
                885                 890                 895

Val Asn Ile Glu Phe Gly Ser Asp Gln Ser Gly Tyr Leu Ser Gly Pro
            900                 905                 910

Val Ala Phe His Ser Ser Pro Ser Val Arg Cys Pro Ser Gln Leu Gln
        915                 920                 925

Pro Ala Pro Arg Glu Glu Glu Thr Gly Thr Glu Glu Tyr Met Lys Met

930                 935                 940

Asp Leu Gly Pro Gly Arg Arg Ala Ala Trp Gln Glu Ser Thr Gly Val
945                 950                 955                 960

Glu Met Gly Arg Leu Gly Pro Ala Pro Pro Gly Ala Ala Ser Ile Cys
                965                 970                 975

Arg Pro Thr Arg Ala Val Pro Ser Ser Arg Gly Asp Tyr Met Thr Met
            980                 985                 990

Gln Met Ser Cys Pro Arg Gln Ser  Tyr Val Asp Thr Ser  Pro Ala Ala
        995                 1000                 1005

Pro Val  Ser Tyr Ala Asp Met  Arg Thr Gly Ile Ala  Ala Glu Glu
    1010                 1015                 1020

Val Ser  Leu Pro Arg Ala Thr  Met Ala Ala Ala Ser  Ser Ser Ser
    1025                 1030                 1035

Ala Ala  Ser Ala Ser Pro Thr  Gly Pro Gln Gly Ala  Ala Glu Leu
    1040                 1045                 1050

Ala Ala  His Ser Ser Leu Leu  Gly Gly Pro Gln Gly  Pro Gly Gly
    1055                 1060                 1065

Met Ser  Ala Phe Thr Arg Val  Asn Leu Ser Pro Asn  Arg Asn Gln
    1070                 1075                 1080

Ser Ala  Lys Val Ile Arg Ala  Asp Pro Gln Gly Cys  Arg Arg Arg
    1085                 1090                 1095

His Ser  Ser Glu Thr Phe Ser  Ser Thr Pro Ser Ala  Thr Arg Val
    1100                 1105                 1110

Gly Asn  Thr Val Pro Phe Gly  Ala Gly Ala Ala Val  Gly Gly Gly
    1115                 1120                 1125

Gly Gly  Ser Ser Ser Ser Ser  Glu Asp Val Lys Arg  His Ser Ser
    1130                 1135                 1140

Ala Ser  Phe Glu Asn Val Trp  Leu Arg Pro Gly Glu  Leu Gly Gly
    1145                 1150                 1155

Ala Pro  Lys Glu Pro Ala Lys  Leu Cys Gly Ala Ala  Gly Gly Leu
    1160                 1165                 1170

Glu Asn  Gly Leu Asn Tyr Ile  Asp Leu Asp Leu Val  Lys Asp Phe
    1175                 1180                 1185

Lys Gln  Cys Pro Gln Glu Cys  Thr Pro Glu Pro Gln  Pro Pro Pro
    1190                 1195                 1200

Pro Pro  Pro Pro His Gln Pro  Leu Gly Ser Gly Glu  Ser Ser Ser
    1205                 1210                 1215

Thr Arg  Arg Ser Ser Glu Asp  Leu Ser Ala Tyr Ala  Ser Ile Ser
    1220                 1225                 1230

Phe Gln  Lys Gln Pro Glu Asp  Arg Gln
    1235                 1240

## Description

La présente invention a pour objet des oligonucléotides antisens capables d'inhiber l'expression de la protéine IRS-1 et d'inhiber la formation des tubes capillaires par les cellules endothéliales. Les oligonucléotides selon l'invention sont donc indiqués comme agent anti-angiogénique. Ils sont également indiqués comme agents anti-multiplication cellulaire, en particulier comme agents anti-tumoraux.

L'invention concerne aussi des compositions pharmaceutiques contenant lesdits oligonucléotides et l'utilisation desdits oligonucléotides comme réactifs d'analyse.

L'angiogénèse est un processus fondamental par lequel de nouveaux vaisseaux sanguins se forment. Ce processus est essentiel dans plusieurs phénomènes physiologiques normaux tels que la reproduction, le développement ou encore la cicatrisation. Dans ces phénomènes biologiques normaux, l'angiogénèse est sous contrôle strict, c'est-à-dire qu'elle est déclenchée pendant une période courte (quelques jours), puis complètement inhibée. Cependant, plusieurs pathologies sont liées à une angiogénèse invasive et incontrôlée; l'arthrite, une pathologie due à l'endommagement des cartilages par les néovaisseaux invasifs; la rétinopathie diabétique où l'invasion de la rétine par les néovaisseaux conduit à l'aveuglement des malades; la néovascularisation de l'appareil oculaire présente la cause majeure de l'aveuglement, et cette néovascularisation intervient dans une vingtaine de maladies de l'oeil; ou encore la croissance et la métastase des tumeurs qui sont liées directement à la néovascularisation et sont dépendantes de l'angiogénèse. La tumeur stimule la croissance des néovaisseaux pour sa croissance elle-même. De plus, ces néovaisseaux sont des voies d'échappement des tumeurs qui joignent ainsi la circulation sanguine et provoquent des métastases dans des sites éloignés du foyer tumoral initial, comme le foie, le poumon ou l'os.

La formation de néovaisseaux par les cellules endothéliales, l'angiogénèse, implique la migration, la croissance, et la différentiation des cellules endothéliales. La régulation de ces phénomènes biologiques est directement liée à l'expression génétique.

Ainsi, les travaux de recherche réalisés dans le cadre de la présente invention ont permis d'identifier et de préparer des séquences d'acide nucléique impliquées dans la régulation de l'angiogénèse.

D'autres travaux concernant l'angiogénèse ont permis de mettre en évidence une expression importante et une phosphorylation, au niveau d'un résidu tyrosine d'une protéine intracellulaire de 180 kDa, par les cellules endothéliales cultivées sur un tapis du collagène type I et stimulées par un facteur angiogénique tel que le bFGF. L'expression importante et la phosphorylation au niveau du résidu tyrosine de la protéine intracellulaire de 180 kDa accompagnent la formation des tubes capillaires par les cellules endothéliales.

Cette protéine est déjà connue en tant que substrat du récepteur de l'insuline (nommé IRS-1). Elle a été, en effet, partiellement identifiée et étudiée par certains auteurs travaillant sur le diabète (Quon et al., J. Biol. Chem. (1994), 269 (45), 27920-27924).

Ces auteurs ont étudié le rôle de l'IRS-1 sur (i) la translocation du GLUT 4 stimulée par l'insuline et (ii) le transport du glucose, dans des cellules adipeuses de rat. Pour ce faire, ils ont construit un plasmide contenant :
- un oligonucléotide bicaténaire, obtenu à partir de l'oligonucléotide sens de séquence SEQ ID No. 1 suivante : 5'-TCGATGTGAC GCTACTGATG AGTCCGTGAG GACGAAACTC TGGCCTAG-3', et
- l'ADNc codant pour l'IRS-1 humain,
et ont transfecté des cellules adipeuses de rat avec ledit plasmide.

Les travaux menés dans le cadre de la présente invention ont mis en évidence le fait que l'expression de la protéine IRS-1 est également induite dans les cellules endothéliales lorsque ces dernières sont stimulées par le facteur angiogénique bFGF.

L'invention concerne donc une composition pharmaceutique active sur les phénomènes d'angiogénèse comprenant à titre d'agent actif au moins une substance choisie parmi : (i) une molécule d'acide nucléique du gène codant pour la protéine IRS-1, d'une séquence complémentaire ou d'un fragment de celle-ci ou un fragment de celle-ci, (ii) une molécule capable d'inhiber l'expression d'une molécule d'acide nucléique selon (i).

Dans le cadre de l'invention, des oligonucléotides anti-sens du gène codant pour cette protéine ont été préparés. Ces oligonucléotides présentent des activités anti-angiogénique et anti-tumorale remarquables. Ils sont donc particulièrement utiles pour le traitement des maladies liées à une angiogénèse invasive et non contrôlée par des méthodes de thérapies géniques consistant à administrer à un individu une composition contenant au moins un de ces oligonucléotides.

Ainsi, un oligonucléotide selon l'invention est constitué par la séquence nucléotidique de formule SEQ ID No.2 suivante :
5'-TATCCGGAGGGCTCGCCATGCTGCTGCGGAGCAGA-3',
un fragment de celle-ci comprenant au moins 12 nucléotides contigus, ou leur dérivé.

L'invention concerne tout particulièrement un oligonucléotide constitué par l'une des séquences nucléotidiques de formules SEQ ID No.3 et 4 suivantes :
5'-TATCCGGAGGGCTCGCCATGCTGCT-3' ,
5'-TCGCCATGCTGCTGCGGAGCAGA-3',
un fragment de celles-ci comprenant au moins 12 nucléotides contigus, ou leur dérivé.

On entend par dérivé, une séquence capable de s'hybrider dans des conditions strictes avec l'une des séquences SEQ ID No.2, 3 ou 4 ou avec un fragment de celles-ci d'au moins 12 nucléotides contigus.

A titre d'exemples d'oligonucléotides selon l'invention, on peut mentionner ceux de séquences suivantes :
SEQ ID No.5: 5'-TATCCGGAGGGCCTGCCATGCTGCT-3'
SEQ ID No.6: 5'-TATCCGGAGG GCCTGCCATG CTGC-3'
SEQ ID No.7: 5'-TATCCGGAGG GCCTGCCATG CTG-3'
SEQ ID No.8: 5'-TATCCGGAGG GCCTGCCATG CT-3'
SEQ ID No.9: 5'-TATCCGGAGG GCCTGCCATG C-3'
SEQ ID No.10: 5'-TATCCGGAGG GCCTGCCATG-3'
SEQ ID No.11: 5'-TATCCGGAGG GCCTGCCAT-3'
SEQ ID No.12: 5'-TATCCGGAGG GCCTGCCA-3'
SEQ ID No.13: 5'-TATCCGGAGG GCCTGCC-3'
SEQ ID No.14: 5'-TATCCGGAGG GCCTGC-3'
SEQ ID No.15: 5'-TATCCGGAGG GCCTG-3'
SEQ ID No.16: 5'-TATCCGGAGG GCCT-3'
SEQ ID No.17: 5'-TATCCGGAGG GCC-3'
SEQ ID No.18: 5'-TATCCGGAGG GC-3'
SEQ ID No.19: 5'-CCGGAGG GCCTGCCATG CTGCT-3'
SEQ ID No.20: 5'-GAGG GCCTGCCATG CTGCT-3'
SEQ ID No.21: 5'-G GCCTGCCATG CTGCT-3'
SEQ ID No.22: 5'-CTGCCATG CTGCT-3'
SEQ ID No.23: 5'-TGCCATG CTGCT-3'

Avantageusement, tout ou partie des liaisons phosphodiester des oligonucléotides de l'invention sont protégées. Cette protection s'effectue généralement par voie chimique, selon des méthodes classiques bien connues de l'homme du métier. Par exemple, on peut protéger les liaisons phosphodiester par une fonction thiol ou amine ou bien encore par un groupement phényle.

De façon également aventageuse, les extrémités 5'- et/ou 3'- des oligonucléotides de l'invention sont protégées, par exemple en utilisant la technique indiquée précédemment pour protéger les liaisons phosphodiester.

Les oligonucléotides de l'invention peuvent être synthétisés selon les techniques conventionnelles bien connues de l'homme du métier, par exemple à l'aide d'un synthétiseur d'ADN commercialisé par différentes sociétés.

Bien que leur mécanisme d'action ne soit pas entièrement élucidé, les oligonucléotides selon l'invention inhibent l'expression de la protéine IRS-1 au sein des cellules endothéliales. Ces oligonucléotides sont capables de bloquer la formation des néovaisseaux par les cellules endothéliales (i.e. inhibent l'angiogénèse), et de ce fait ils inhibent la multiplication des cellules tumorales chez la souris.

L'invention a donc aussi pour objet une composition pharmaceutique pour l'inhibition du gène codant pour la protéine IRS-1 comprenant au moins un oligonucléotide complémentaire d'une partie dudit gène ou d'un transcrit dudit gène.

De préférence, la molécule capable d'inhiber l'expression d'une molécule d'acide nucléique du gène codant pour la protéine IRS-1 est une séquence antisens de la région codante de la séquence identifiée sous le numéro SEQ ID No. 28 dans la liste de séquences en annexe.

Avantageusement, ladite séquence antisens comprend au moins douze nucléotides contigus ou leur dérivé.

Plus préférentiellement, l'agent actif capable d'inhiber l'expression d'une molécule d'acide nucléique du gène codant pour la protéine IRS-1 de la composition de l'invention est une séquence nucléotidique choisie parmi l'ensemble de séquences nucléotidiques identifiées sous les numéros SEQ ID No. 2 à SEQ ID No 23 dans la liste de séquences en annexe comprenant au moins douze nucléotides contigus ou leur dérivé.

Une telle composition comprend avantageusement comme agent actif au moins un oligonucléotide défini précédemment, avantageusement associé dans ladite composition avec un véhicule acceptable.

L'analyse des travaux réalisés dans le cadre de l'invention a donc permis de montrer que la protéine IRS-1 représente un constituant cellulaire essentiel dans le processus de l'angiogénèse. En effet, l'inhibition de l'expression de la protéine IRS-1 par lesdits oligonucléotides antisens conduit à l'inhibition de la formation de tubes capillaires par les cellules endothéliales.

Les oligonucléotides selon l'invention et les compositions les contenant sont donc indiqués comme agent anti-angiogénique. Ils sont également indiqués comme agents anti-multiplication cellulaire, en particulier comme agents anti-tumoraux, et en conséquence sont tout particulièrement utiles pour le traitement des tumeurs. L'invention a donc pour objet l'utilisation desdits oligonucléotides pour la préparation d'une composition destinée au traitement ou à la prévention de pathologies liées à une angiogénèse invasive et incontrôlée, comme par exemple à titre non limitatif: le traitement de la vascularisation de tumeurs, des maladies de l'oeil liées à la néovascularisation de l'appareil oculaire telles que les rétinopathies, l'arthrite rhumatoïde, la maladie de Crohn, l'athérosclérose, l'hyperstimulation de l'ovaire, le psoriasis, l'endométrie associée à la néovascularisation, la resténose due à l'angioplastie du ballon, la superproduction tissulaire due à la cicatrisation, la maladie vasculaire périphérique, l'hypertension, l'inflammation vasculaire, la maladie et les phénomènes de Raynaud, l'anévrisme, la resténose artérielle, la thrombophlébite, la lymphagyte, le lymphodème, la cicatrisation et la réparation tissulaire, l'ischémie, l'angine, l'infarctus de myocarde, la maladie chronique du coeur, les insuffisances cardiaques telles que l'insuffisance cardiaque congestive, la dégénération maculaire liée à l'âge et l'ostéoporose.

Les compositions pharmaceutiques ci-dessus sont plus particulièrement réalisées de manière à pouvoir être administrées par voie sous-cutanée, intramusculaire, intraveineuse ou transdermique. Pour une telle administration, on utilise notamment des suspensions aqueuses, des solutions salines isotoniques ou des solutions stériles et injectables qui contiennent des agents de dispersion et/ou des agents mouillants pharmacologiquement compatibles, par exemple le propylèneglycol ou le butylèneglycol.

La dose unitaire usuelle à administrer contient de 0,001 mg à 50 mg de principe actif.

Les oligonucléotides de l'invention sont aussi utiles comme réactif de recherche, notamment pour l'étude *in vitro* des voies de signalisation impliquant la protéine 180 kDa, par exemple sur des cellules, tumorales ou non, transfectées par lesdits oligonucléotides. Ils sont également utiles pour l'étude *in vivo* des voies de signalisation impliquant la protéine 180 kDa dans un grand nombre de phénomènes physiologiques et pathologiques, comme l'angiogénèse ou la cancérogenèse, essentiellement à partir du rapport kinase/phosphatase.

Ainsi, les composition pharmaceutiques de l'invention sont particulièrement utiles pour la mise en oeuvre des tests de diagnostic des pathologies liées aux phénomènes d'angiogénèse, notamment pour le diagnostic des rétinopathies, de l'arthrite rhumatoïde, de la maladie de Crohn, de l'athérosclérose, l'hyperstimulation de l'ovaire, du psoriasis, de l'endométrie associée à la néovascularisation, de la resténose due à l'angioplastie du ballon, de la superproduction tissulaire due à la cicatrisation, de la maladie vasculaire périphérique, de l'hypertension, de l'inflammation vasculaire, de la maladie et les phénomènes de Raynaud, de l'anévrisme, de la resténose artérielle, de la thrombophlébite, de la lymphagyte, du lymphodème, de la cicatrisation et de la réparation tissulaire, de l'ischémie, de l'angine, de l'infarctus de myocarde, de la maladie chronique du coeur, des insuffisances cardiaques telles que l'insuffisance cardiaque congestive ou de la dégénération maculaire liée à l'âge et de l'ostéoporose.

D'autres avantages et caractéristiques de l'invention apparaîtront des exemples qui suivent dans lesquels on désigne par "oligonucléotide" l'oligonucléotide de séquence ID NO.3 et qui font référence aux figures jointes annexe dans lesquelles :
- la figure 1A représente l'image du Western Blot obtenu à partir des échantillons de surnageants issus des cellules non stimulées (Piste NS) et des cellules stimulées avec le bFGF (Piste S), révélée par un anticorps anti-IRS-1.
- la figure 1B montre l'image du Western blot obtenue après coloration au nitrate d'argent à partir des mêmes échantillons de surnageants issus des cellules non stimulées (Piste NS) et des cellules stimulées avec le bFGF (Piste S)
- la figure 2 montre les images du Western Blot obtenues à partir des échantillons des surnageants issus des cellules non stimulées (Piste NS) et des cellules stimulées avec le bFGF (Piste B), lorsque la membrane est incubée avec un anticorps monoclonal anti-phosphotyrosine et révélée avec un anticorps anti-isotype marqué à la peroxydase comme indiqué dans l'exemple 3,

- Les figures 3A à 3D montrent les images des cultures, sur un tapis du collagène type I, des différents lots de cellules endothéliales :

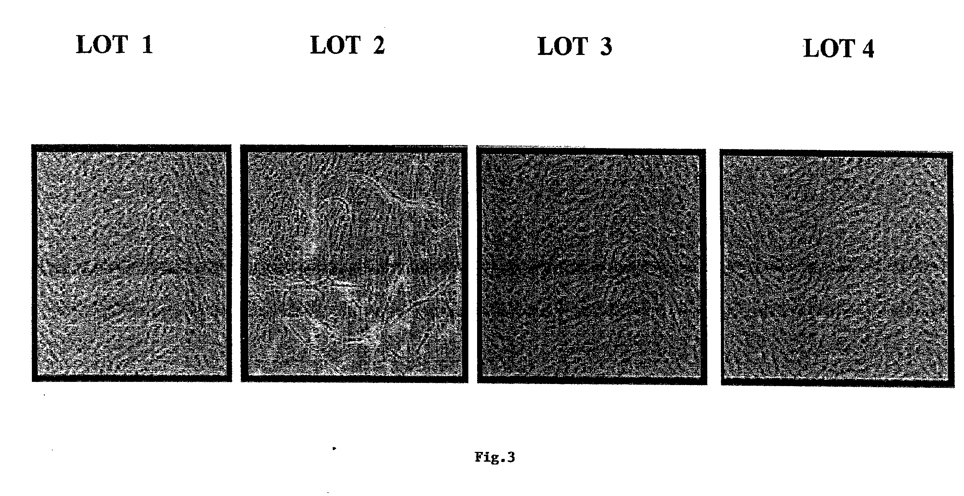

   - La figure 3A montre la culture des cellules endothéliales non traitées.
   - La figure 3B montre la culture des cellules endothéliales stimulées avec 3 ng/ml de bFGF.
   - la figure 3C montre la culture des cellules endothéliales incubés avec 100 µg/ml d'oligonucléotide de séquence SEQ ID No. 3 pendant 4 heures, puis stimulées avec 3 ng/ml de bFGF.
   - la figure 3D montre la culture des cellules endothéliales incubées avec 100 µg/ml de l'oligonucléotide de séquence SEQ ID No. 3 pendant 4 heures.

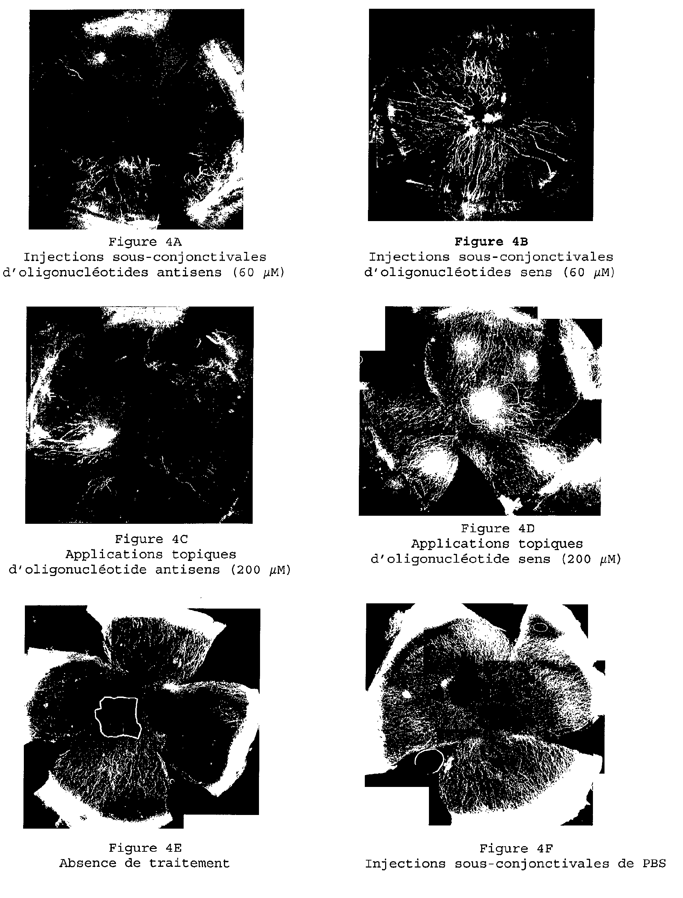

- Les figures 4A à 4F illustrent les résultats des essais d'inhibition de la néovascularisation cornéenne obtenue chez des rats.
   La figure 4A montre les résultats obtenus par injection sous-conjonctivale d'un oligonucléotide antisens à une concentration de 60 µm.
   La figure 4B illustre les résultats obtenus après injection sous-conjonctivale d'un oligonucléotide sens à une concentration de 60 µM.
   La figure 4C montre les résultats obtenus après une application topique d'un oligonucléotide antisens à une concentration de 200µM.
   La figure 4D montre les résultats obtenus après une application topique d'un oligonucléotide sens à une concentration de 200µM.
   La figure 4E illustre l'état de la cornée en absence de tout traitement.
   La figure 4F montre l'état de la cornée lorsque celle-ci est traitée avec des injonctions sous-conjonctivales de PBS.

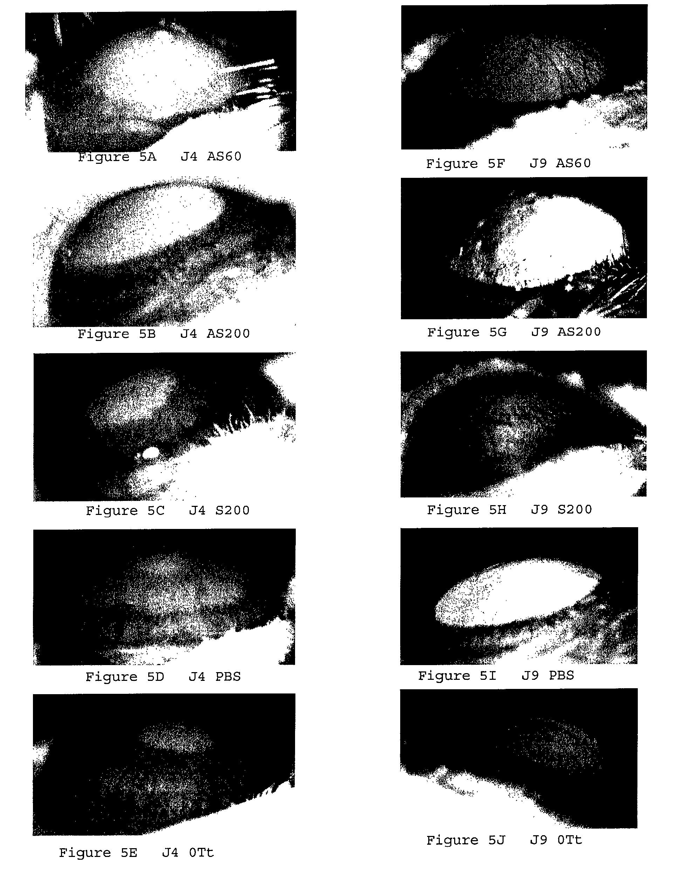

Les figures 5A à 5J illustrent les résultats de l'inhibition de la néovascularisation cornéenne obtenus chez les différents groupes de rats (décrits section 6.6 Résultats), après désépithélialisation et résection limbique des cornées de rats à jour 4 (Figures 5A à 5E) et à jour 9 (Figures 5F à 5J). Il s'agit de photographies en lampe à fente montrant la comparaison de la croissance des vaisseaux dans les différents groupes de rats. Grossissement x10

### Exemple 1 : Mise en évidence de l'induction de l'expression d'IRS-1 (la protéine de 180 kDa) dans les cellules endothéliales suite à la stimulation de ces cellules par le bFGF.

La protéine 180 kDa a été mise en évidence de la manière suivante :
Les cellules endothéliales ont été mises en culture dans une plaque de microtitration à 6 puits préalablement recouverts avec du collagène type I comme décrit dans (Montesano et al., J. Cell. Biol., 1983, 83, 1648-1652). Le milieu de culture est le DMEM (Sigma) enrichi avec 10 % de sérum de voeu fétal, 4 mM glutamine, 500 U/ml pénicilline, et 100 µg/ml streptomycine. Après 3 à 4 jours de culture, on obtient une couche semi-confluente des cellules endothéliales. Les milieux de culture de six puits ont été aspirés et remplacés par du milieu de culture frais. Trois puits ont été enrichis avec 3 ng/ml de bFGF. Après une incubation de 48 heures, les différents puits sont lavés trois fois avec un tampon phosphate, et les cellules sont utilisées pour extraire l'ARN messager (ARNm) selon des protocoles connus de l'homme du métier. Les ARNm sont reverse transcrits par une réaction de polymérisation en chaîne (PCR) en utilisant chacun des quatre groupes dégénérés d'amorces oligo(dT) (T12MN), M peut être G, A, ou C; et N est G, A, T, et C. Chaque groupe d'amorces est dicté par la base en position 3'(N), avec une dégénérescence dans la position (M). Exemple: le set d'amorces où N = G est constitué de:
   SEQ ID No.24: 5'-TTTTTTTTTTTTGG-3'
   SEQ ID No.25: 5'-TTTTTTTTTTTTAG-3'
   SEQ ID No.26: 5'-TTTTTTTTTTTTCG-3'

Les ADNc ainsi obtenus sont amplifiés et marqués au moyen d'un décamère arbitraire, en présence d'ATP marqué isotopiquement. L'analyse des ADNc amplifiés par électrophorèse a révélé la présence d'un fragment d'ADNc de 326 bp amplifié dans l'échantillon issu des cellules endothéliales stimulées avec le bFGF, identifié dans la liste de séquences en annexe sous le numéro SEQ ID.No. : 27, cependant, ce même fragment est faiblement présent ou présent à l'état de trace dans l'échantillon issu des cellules endothéliales non stimulées avec le bFGF. Le séquençage de ce fragment et l'interrogation subséquente des banques des données ont révélé que ce fragment correspond à une partie d'un gène déjà connu, codant pour le substrat du récepteur de l'insuline (une protéine intracellulaire de 180 kDa).

### Exemple 2 : Mise en évidence de l'induction de l'expression d'IRS-1 (la protéine de 180 kDa).

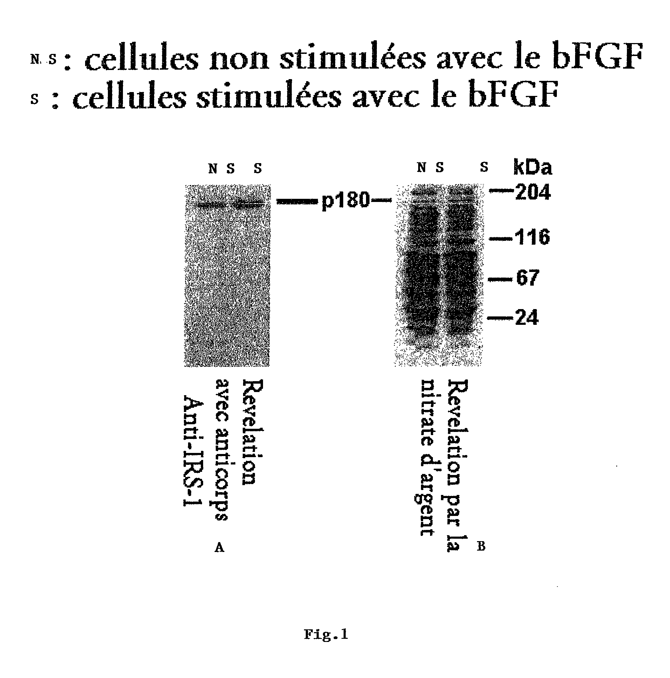

Des cellules endothéliales en culture sur un tapis du collagène type I stimulées ou non stimulées avec le bFGF (cf. exemple 1) sont lysées dans un tampon de lyse cellulaire contenant de l'orthovanadate de sodium. Ces solutions sont ensuite clarifiées par centrifugation à 14000 g pendant 15 min. Des échantillons de surnageants issus des cellules non stimulées et des cellules stimulées avec le bFGF, contenant des quantités équivalentes de protéines, ont été repris avec une solution d'électrophorèse contenant 2% SDS et 15 mM de dithiothréitol, chauffés à 100 °C pendant 5 min. puis déposés en gel de polyacrylamide (gradient de 4 à 15% en acrylamide) dans des conditions dénaturantes (en présence de 2% SDS). Après migration, les protéines sont transférées sur une membrane de nitrocellulose. La membrane est bloquée par une incubation à température ambiante dans une solution de 5% de lait dans un tampon PBS. La membrane est ensuite lavée trois fois avec un tampon PBS, incubée dans un tampon PBS contenant 1 µg/ml d'anticorps monoclonal anti-IRS-1 pendant 2 heures à température ambiante, et lavée trois fois avec un tampon PBS. Les protéines sont ensuite révélées au moyen d'un anticorps secondaire anti-isotype couplé à la peroxydase. On constate la présence d'une protéine de poids moléculaire 180 kDa reconnue par l'anticorps anti-IRS-1 monoclonal dans les préparations issues des cellules endothéliales stimulées avec le bFGF; cette protéine est faiblement présente dans la préparation issue des cellules endothéliales non stimulées avec le bFGF (Figure 1).

### Exemple 3 : Mise en évidence de l'induction de la phosphorylation au niveau de tyrosine d'IRS-1 (la protéine de 180 kDa).

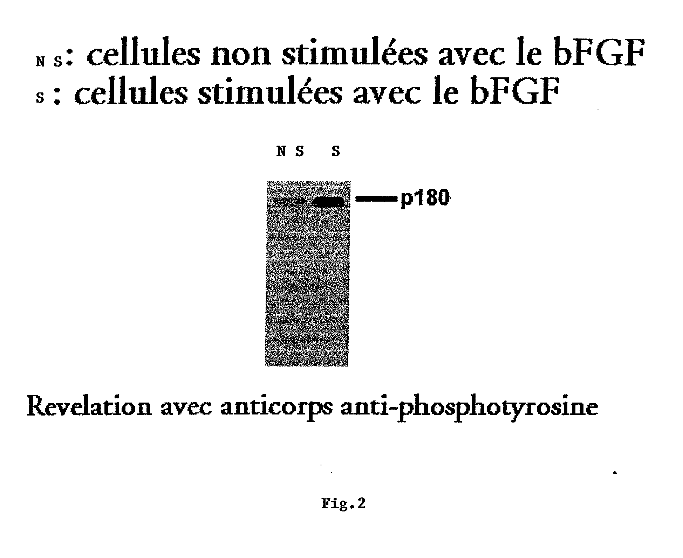

Des cellules endothéliales humaines en culture sur un tapis du collagène type I stimulées ou non stimulées avec le bFGF sont lysées dans un tampon de lyse cellulaire contenant de l'orthovanadate de sodium. Ces solutions sont ensuite clarifiées par centrifugation à 14000 g pendant 15 min (cf. exemple 2). La protéine IRS-1 a été extraite grâce à un anticorps monoclonal anti-IRS-1. Cette extraction est réalisée après une immunoprécipitation à l'aide d'un anticorps monoclonal anti-IRS-1 (Sigma). Après addition de l'anticorps anti-IRS-1 couplé à l'agarose, la suspension est incubée pendant 2 heures à température ambiante, puis centrifugée à 4000 g pendant 15 min. Le précipité obtenu est repris avec une solution d'électrophorèse contenant 2% SDS et 15 mM de dithiothréitol, chauffé à 100 °C pendant 5 min., puis déposé en gel de polyacrylamide (gradient de 4 à 15% en acrylamide) dans des conditions dénaturantes (en présence de 2% SDS). Après migration, les protéines sont transférées sur une membrane de nitrocellulose. La membrane est bloquée par une incubation à température ambiante dans une solution de 5% de lait dans un tampon PBS. La membrane est ensuite lavée trois fois avec un tampon PBS, incubée dans un tampon PBS contenant 1 µg/ml d'anticorps monoclonal anti-phosphotyrosine pendant 2 heures à température ambiante, et lavée trois fois avec un tampon PBS. Les protéines sont ensuite révélées au moyen d'un anticorps secondaire anti-isotype couplé à la peroxydase. On constate que la protéine IRS-1 de poids moléculaire 180 kDa est phosphorylée au niveau du résidu tyrosine dans les préparations issues des cellules endothéliales stimulées avec le bFGF ; cette protéine est très faiblement phosphorylée au niveau du résidu tyrosine dans la préparation issue des cellules endothéliales non stimulées avec le bFGF (Figure 2)

### Exemple 4 : Evaluation de l'activité anti-angiogénique in vitro de l'oligonucléotide.

Des cellules endothéliales humaines sont mises en culture sur un tapis du collagène type I. Au septième jour de culture, les puits de culture sont divisés en quatre lots :
Lot 1 : Puits correspondant à la culture des cellules endothéliales non traitées. (Figure 3A)
Lot 2 : Puits correspondant à la culture des cellules endothéliales stimulées avec 3 ng/ml de bFGF. (Figure 3B)
Lot 3 : Puits correspondant à la culture des cellules endothéliales incubées avec 100 µg/ml d'oligonucléotide de séquence SEQ ID No. 3 pendant 4 heures, puis stimulées avec 3 ng/ml de bFGF. (Figure 3C)
Lot 4 : Puits correspondant à la culture des cellules endothéliales incubées avec 100 µg/ml d'oligonucléotide de séquence SEQ ID No. 3 pendant 4 heures. (Figure 3D)

Après 3 à 4 jours de culture, les différents puits sont examinés à l'aide de microscope optique à phase inverse. A la lecture des résultats, il apparaît que les cellules endothéliales humaines dans le lot 2 forment des tubes capillaires suite à la stimulation avec le bFGF. On constate aussi que l'oligonucléotide inhibe la formation de néovaisseaux par ces mêmes cellules stimulées avec le bFGF dans le lot 3. On observe enfin que l'oligonucléotide ne modifie pas de manière prononcée la croissance des cellules endothéliales. En effet, le nombre des cellules endothéliales dans le puits de lot 1 et ceux de lot 4 sont comparables.

### Exemple 5 : Evaluation de l'activité in vivo de l'oligonucléotide.

Trois lots de souris nude ont été utilisés. Chaque lot étant constitué de 5 souris.

Lot N°1 : Ce lot a servi de témoin. Chaque souris est inoculée en sous-cutané à J0 avec 200 µl d'une suspension de cellules de mélanome B16 (fournies par l'Institut Gustave Roussy de Villejuif) dispersées dans du PBS à raison de 10⁶ cellules/ml. Ces souris ne sont pas traitées par la suite.

Lot N°2 : Chaque souris est inoculée en sous-cutané à J0 avec 200 µl d'une suspension de cellules de mélanome B16 dispersées dans du PBS à raison de 10⁶ cellules/ml. A J1, J2, J3, J4, J5, J6, J7, J8, J9 et J10 chaque souris reçoit une injection en sous-cutanée de 200 µl d'une solution d'oligonucléotide diluée dans du PBS à une concentration de 500 µg/ml. L'injection d'oligonucléotide est effectuée à proximité du site d'injection des cellules.

Lot N°3 : Les souris de ce lot ne sont pas inoculées avec les cellules de mélanome B16. Cependant, chacune de ces souris reçoit une injection de 200 µl d'une solution d'oligonucléotide dans du PBS à une concentration de 500 µg/ml ; les injections sont faites à J1, J2 J3, J4, J5, J6, J7, J8, J9 et J10.

Les résultats sont les suivants :
Après inoculation, chez les souris du lot N°1, la masse tumorale se développe d'une façon très rapide. En effet, la masse tumorale atteint une taille de 1,6 à 2,5 cm de diamètre après dix jours chez les souris dudit lot N°1 (souris non traitées). L'évolution de la masse tumorale chez les souris du lot N°2 (souris traitées après inoculation par injection d'oligonucléotide à J1, J2 et J3), montre une augmentation nettement moindre du volume de la masse tumorale. La masse tumorale chez les souris du lot 2 ne dépasse pas 0,8 cm de diamètre au dixième jour. Au quatorzième jour, la différence entre la masse tumorale des souris de lots N°2 et celle des souris du lot N°1 est remarquable.

Chez les souris du lot N°3 (souris n'ayant pas reçu de cellules de mélanome B16, mais traitées par injection d'oligonucléotide pendant trois jours) on note un effet général inattendu sur la peau. Il est identique à celui noté chez toutes les souris qui ont été traitées par l'oligonucléotide (lot 2). La peau prend un aspect vieilli, fripé. On note aussi l'apparition de poils chez toutes les souris traitées. Il existe un parallélisme au cours de l'évolution entre la régression des signes cutanés et la reprise de la croissance tumorale.

On constate donc que l'oligonucléotide inhibe le développement et la formation des néovaisseaux par les cellules endothéliales *in vitro*; Il possède d'autre part une activité anti-tumorale *in vivo* remarquable chez la souris nude.

### Exemple 6: Evaluation de l'oligonucléotide anti-angiogénique sur un modèle de néovascularisation cornéenne chez le rat

Sur la base des travaux d'Amano et al. (1998), le demandeur a repris, modifié et analysé un modèle de formation de néovaisseaux cornéens chez le rat après désépithélialisation et limbectomie (Figures 5A à 5J ). Il est reproductible, permet d'examiner directement les néovaisseaux en lampe à fente et de les quantifier, les détails sont indiqués ci-dessous. Ce modèle a ensuite été utilisé pour tester l'efficacité d'agents anti-angiogéniques de l'invention.

### 6.1 Animaux et modèle de néovascularisation cornéenne.

Des rats (Rattus norvegicus) Wistar mâles âgés de cinq semaines (Charles River France, St-Aubin les Elbeufs, France), exempts de germes pathogènes spécifiques sont nourris et abreuvés à volonté, et maintenus, dans l'animalerie du laboratoire, dans des conditions fixes de température et d'humidité, avec des cycles de 12 heures de lumière / 12 heures d'obscurité.

Les rats sont anesthésiés avec un mélange de kétamine (Kétamine 1000, UVA, Ivry-sur-Seine, France ; 128 mg/kg) et de chlorpromazine (Largactil 25 mg/ml; Specia Rhône Poulenc, Paris, France; 5 mg/kg), injecté par voie intra-musculaire. Une goutte d'oxybuprocaïne (Novésine, Chibret, Clermont-Ferrand, France) est instillée dans l'oeil droit. A l'aide d'un système grossissant (macroscope Wild MPS 51 S, LEICA, Heerbrugg, Suisse), l'épithélium cornéen est enlevé par une « microsponge » imbibée d'éthanol à 70%. Une bande de conjonctive de 1,5 mm de large, au limbe, est excisée à l'aide de ciseaux microchirurgicaux, et les paupières sont fermées par une blépharorraphie temporaire à l'aide d'un fil Vicryl 5.0 (Dacron, Alcon, Rueil-Malmaison, France). L'oeil est ensuite rincé abondamment au PBS 1X, une pommade oxytétracycline (Posicycline, Alcon, France) est appliquée et la blépharorraphie est ouverte le quatrième jour (8, 9).

### 6.2 Traitement par injections sous-conjonctivales et applications topiques de l'oligonucléotide anti-angiogénique.

L'ensemble de rats a été divisé en 6 groupes :
Groupe A : modèle + injection sous-conjonctivale d'une solution d'oligonucléotide antisens à 60 µM dans du PBS 1X,
Groupe B : modèle + application topique d'une solution d'oligonucléotide antisens à 200 µM dans du PBS 1x,
Groupe C : modèle + injection sous-conjonctivale d'une solution d'oligonucléotide sens à 60 µM dans du PBS 1X,
Groupe D : modèle + application topique d'une solution d'oligonucléotide sens à 200 µM dans du PBS 1X,
Groupe E : modèle + injection sous-conjonctivale de PBS 1X,
Groupe F : modèle sans traitement).

Tous les rats ont été désépithélialisés comme décrit ci-dessus ; le traitement a été effectué toutes les 24 heures à partir du quatrième jour, et jusqu'au neuvième jour. La néovascularisation a été examinée en début, milieu et fin de protocole, par examen en lampe à fente ; des photographies ont été prises à J0 et J9.

### 6.3 Visualisation et quantification de la néovascularisation.

Les animaux ont été euthanasiés 10 jours après la désépithélialisation, par injection létale de pentobarbital (injection intra-péritonéale). Pour remplir les micro-vaisseaux et quantifier la néovascularisation cornéenne, la partie supérieure du corps de l'animal a été perfusée avec de la Fluorescéine-Dextran 2x1 000 000. Les yeux ont été énucléés et immergés dans du paraformaldéhyde/PBS 1X 4% pendant 3 heures, puis une nuit dans du PBS 1X. On a ensuite isolé la cornée avec 1 mm de limbe, sous microscope opératoire, et on l'a mise à plat entre lame et lamelle grâce à 3 à 5 incisions radiaires. Les cornées à plat ont ensuite été examinées et photographiées en microscopie à fluorescence. Une fois les cornées entières reconstituées, on les a scannées puis on a mesuré les surfaces par analyse d'image ; un logiciel (NIH image) permettant la quantification de la néovascularisation. Pour chaque photo, on a mesuré 3 fois la surface cornéenne totale, et 3 fois la surface néovascularisée ; le rapport des moyennes - surface néovascularisée / surface cornéenne totale - nous permet d'obtenir le « pourcentage de néovascularisation », et de mesurer l'inhibition obtenue.

### 6.4 Analyse statistique

Les résultats sont exprimés en moyennes ± SD. Les pourcentages de surface néovascularisée/ surface totale ont été comparés avec le test non paramétrique de Mann-Whitney. Les valeurs de P<0,05 sont considérées comme significatives.

### 6.5 Dilution de l'oligonucléotide

L'oligonucléotide est dilué dans du PBS 1X à pH 7,2. En fonction des données de la littérature et des expériences menées par ailleurs avec d'autres oligonucléotides, il a été choisi d'utiliser une concentration de 60 µM pour les injections sous-conjonctivales, et une concentration de 200 µM pour les applications topiques.

### 6.6 Résultats

Sur le modèle de néovaisseaux cornéens, il a été effectué un traitement avec l'oligonucléotides 5'-TATCCGGAGGGCTCGCCATGCTGCT-3', identifié sous le numéro SEQ IQ No. 3 dans la liste de séquences en annexe, modifié sous forme phosphorothioate, par jour, de J4 à J9, selon le protocole suivant :
Groupe A : injection sous-conjonctivale de l'oligonucléotide antisens à 60 µM (AS 60)
Groupe B : application topique de l'oligonucléotide antisens à 200 µM (AS 200)
Groupe C : injection sous-conjonctivale de l'oligonucléotide sens à 60 µM (S 60)
Groupe D : application topique de l'oligonucléotide sens à 200 µM (S 200)
Groupe E : injection sous-conjonctivale de PBS 1X (PBS)
Groupe F : pas de traitement (O Tt)

Le dixième jour du protocole, les rats ont été perfusés avec une solution de FITC/Dextran, puis euthanasiés, les cornées ont été prélevées et fixées dans une solution de PAF 4%; ensuite les cornées ont été mises à plat entre lame et lamelle dans une solution de glycérol; les néovaisseaux fluorescents ont été observés et photographiés au microscope à fluorescence, les photos ont été scannées, et mesuré les pourcentages de néovascularisation pour chaque animal.

Les résultats observés sont présentés dans le tableau 1 ci-dessous :

**Tableau 1**

| | **Groupe** **A** **AS 60** | **Groupe** **B** **AS 200** | **Groupe** **C** **S 60** | **Groupe** D **S 200** | **Groupe** **E** **PBS** | **Groupe** **F** **O Tt** |
|---|---|---|---|---|---|---|
| Moyenne | 0,6157 | 0,5058 | 0,9431 | 0,9392 | 0,9552 | 0,9170 |
| SD | 0,2194 | 0,1172 | 0,0964 | 0,0308 | 0,0481 | 0,0751 |
| Nombre de mesures | 15 | 15 | 15 | 12 | 9 | 9 |
| SEM | 0,0566 | 0,0303 | 0,0249 | 0,0089 | 0,0160 | 0,0250 |

L'analyse statistique des résultats par un test non paramétrique de Mann-Whitney donne les résultats suivants :
Les injections sous-conjonctivales d'ODN antisens à 60 µM (A) ont réduit la néovascularisation par rapport aux groupes E et F témoins (résultats très significatifs, P<0,0001 et P=0,0011) ; l'application topique de l'oligonucléotide antisens à une concentration de 200 µM (B) a réduit la néovascularisation par rapport aux groupes témoins E et F (résultats extrêmement significatifs, P<0,0001).

Comparées à l'administration sous-conjonctivale de d'ODN sens à 60 µM (C) ou à l'application topique d'ODN sens à 200 µM (D), l'injection d'ODN antisens à 60 µM (A) et l'application topique d'ODN antisens à 200 µM (B) ont réduit la néovascularisation. Ces résultats sont extrêmement significatifs P<0,0001. (Figures 4A à 4F).

L'inhibition de la néovascularisation n'est pas significativement différente, selon que l'on administre l'oligonucléotide antisens par voie sous-conjonctivale (60 µM) ou par voie topique (200 µM) ; elle est d'environ 35% par rapports aux témoins (E et F).

L'injection sous-conjonctivale d'ODN sens à 60 µM (C) et l'application topique d'ODN sens à une concentration de 200 µM (D) n'ont pas modifié la néovascularisation par rapport aux groupes témoins (E et F). Par contre, on observe un petit effet de l'oligonucléotide sens en application topique (D) par rapport à l'oligonucléotide sens en injections sous-conjonctivales (C) (P=0,0117).

Par ailleurs, on observe, dans les groupes traités par l'antisens (A et B), un calibre et une densité moindres des néovaisseaux. Leur répartition n'est pas différente par rapport aux groupes témoins, et aucune différence de niveau d'inflammation n'est observée (Figure 4).

### 6.7 Effets secondaires

Au cours des deux séries d'expériences, aucun effet secondaire notable n'a été observé dans aucun groupe : après 6 jours de traitement aux doses mentionnées ci-dessus, la peau des rats n'était pas fripée, le pelage ne s'est pas modifié, l'état général des animaux était bon ; ils se sont nourris normalement jusqu'au dernier jour, et aucune mortalité suspecte n'a été observée. Bien qu'il n'ait été pratiqué ni autopsie, ni analyse sanguine, l'état général des animaux en fin d'expérimentation ne laisse pas supposer l'existence de troubles hépatiques. Le seul symptôme observé est un dépôt blanchâtre transitoire, à l'endroit des injections sous-conjonctivales, chez 60% des rats du groupe A, 60% des rats du groupe C, et 10% des rats du groupe E. Ce dépôt s'est résorbé en fin d'expérimentation dans tous les cas.

Cet exemple montre que, contrairement à ce qu'on pouvait attendre, les injections sous-conjonctivales d'oligonucléotide antisens à une concentration de 60µM n'ont pas plus inhibé la néovascularisation que l'administration topique d'oligonucléotide antisens à une concentration de 200µM.

Ceci peut s'expliquer en partie par la différence des concentrations utilisées ; mais ce résultat suggère également une pénétration de l'oligonucléotide par voie topique plutôt que par le limbe. Il suggère également l'absence de libération prolongée du produit à partir du site d'injection.

### 6.8 Conclusion

L'application de l'oligonucléotide antisens, par voie topique ou en injections sous-conjonctivales réduit la néovascularisation sur notre modèle de néovaisseaux cornéens chez le rat.
Le but de ce travail était de tester l'efficacité des oligonucléotides anti-sens issus de la séquence du gène IRS-1 sur un modèle de néovascularisation cornéenne chez le rat préalablement mis au point.
Ce modèle est facilement accessible, reproductible, et quantifiable. De plus, cette étude a permis une première évaluation de la concentration d'oligonucléotide nécessaire, *in vivo*, pour inhiber une néovascularisation.

### BIBLIOGRAPHIE

1-Aiello LP. Keeping in touch with angiogenesis. Nat Med 2000 ; 6 : 379-381
2-D'Amore P.A. Mechanisms of Retinal and Choroidal Neovascularization. Invest Ophthalmol Vis Sci 1994; 35(12) : 3974-3979
3-Hélène C. Rational design of sequence-specific oncogene inhibitors based on antisense and antigene oligonucleotides. Eur J Cancer 1991 ; 27 : 1466-1471
4-Agrawal S, Bunnel BA, Crooke ST, Davidkova G, Gyurko R, Iyer K et al. Antisense oligonucleotides and antisense RNA. Benjamin Weiss edition (Philadelphie, USA) 1997 ; 1-11, 19-40
5-Pierga JY, Cammilleri S, Benyahia B, Magdelénat H. Applications des oligonucléotides antisens en cancérologie. Bull Cancer 1994 ; 81 : 1023-1042
6-Robinson GS, Pierce EA, Rook SL, Foley E, Webb R, Smith LEH. Oligodeoxynucleotides inhibit retinal neovascularization in a murine model of proliferative retinopathy. Proc Natl Acad Sci USA 1996 ; 93 : 4851-4856
7-Aiello LP. Vascular endothelial growth factor. Invest Ophthalmol Vis Sci 1997 ; 38 :1647-1652
8-Amano S, Rohan R, Kuroki M, Tolentino M, Adamis AP. Requirement for vascular endothelial growth factor in wound and inflammation-related corneal neovascularization. Invest Ophthalmol Vis Sci 1998 ; 39 : 18-22
9-Hoang-Xuan T, Prisant O. Restauration de l'épithélium cornéen à partir des cellules souches limbiques. Med Sci 1998 ; 14 : 1375-1377
10-Parry T.J, Cushman C, Gallegos A.M, Agrawal A.B, Richardson M, Andrews L.E. et al. Bioactivity of antiangiogenic ribozymes targeting Flt-1 and KDR mRNA. Nucleic Acids Research 1999; 27: 2569-2577
11-Ozaki H, Seo MS, Ozaki K, Yamada H, Yamada E, Okamoto N et al. Blockade of vascular endothelial cell growth factor receptor signaling is sufficient to completely prevent retinal neovascularization. Am J Pathol 2000; 156: 697-707
12-Berdugo Polak M, Administration par iontophorèse d'oligonucleotides antisens dans le segment antérieur de l'oeil : application à un modèle de néovascularisation cornéenne chez le rat. DEA "Biologie et Pathologie des Epithéliums"; université Paris VII, Feldmann G.; Inserm U450, directeur Courtois Y., sous la direction de Behar Cohen F. 2000

## Revendications

1. Composition pharmaceutique active sur les phénomènes d'angiogénèse comprenant à titre d'agent actif au moins une substance choisie parmi : (i) une molécule d'acide nucléique du gène codant pour la protéine IRS-1, d'une séquence complémentaire ou d'un fragment de celle-ci, (ii) une molécule capable d'inhiber l'expression d'une molécule d'acide nucléique selon (i).

2. Composition pharmaceutique selon la revendication 1, **caractérisée en ce qu'**elle comprend à titre d'agent actif au moins une séquence nucléotidique choisie parmi l'ensemble de séquences nucléotidiques identifiées sous les numéros SEQ ID No. 2 à SEQ ID No 23 et SEQ ID No. 28 dans la liste de séquences en annexe.

3. Composition pharmaceutique selon la revendication 1 ou 2, **caractérisée en ce que** la molécule capable d'inhiber l'expression d'une molécule d'acide nucléique du gène codant pour la protéine IRS-1 est une séquence antisens de la région codante de la séquence identifiée sous le numéro SEQ ID No. 28 dans la liste de séquences en annexe comprenant au moins douze nucléotides contigus ou leur dérivé.

4. Composition pharmaceutique selon l'une des revendications 1 à 3, **caractérisée en ce que** la molécule capable d'inhiber l'expression d'une molécule d'acide nucléique du gène codant pour la protéine IRS-1 est choisie parmi le groupe comprenant les séquences SEQ ID NO.2 à SEQ ID NO. 23 dans la liste de séquences en annexe, ou un fragment de celles-ci comprenant au moins douze nucléotides contigus, ou leur dérivé.

5. Composition pharmaceutique selon l'une quelconque des revendications 1 à 4 dans laquelle l'agent actif est avantageusement associé dans ladite composition avec un véhicule acceptable.

6. Composition pharmaceutique selon l'une quelconque des revendications 1 à 5, **caractérisée en ce qu'**elle se présente sous une forme pour être administrée par voie sous-cutanée, intramusculaire, intraveineuse ou transdermique, et **en ce qu'**elle comprend de 0,001 mg à 50 mg d'agent actif.

7. Utilisation d'une composition pharmaceutique selon l'une quelconque des revendications 1 à 6 pour le traitement des pathologies liées à l'angiogénèse.

8. Utilisation selon la revendication 7 pour le traitement des rétinopathies, l'arthrite rhumatoïde, la maladie de Crohn, l'athérosclérose, l'hyperstimulation de l'ovaire, le psoriasis, l'endométrie associée à la néovascularisation, la resténose due à l'angioplastie du ballon, la superproduction tissulaire due à la cicatrisation, la maladie vasculaire périphérique, l'hypertension, l'inflammation vasculaire, la maladie et les phénomènes de Raynaud, l'anévrisme, la resténose artérielle, la thrombophlébite, la lymphagyte, le lymphodème, la cicatrisation et la réparation tissulaire, l'ischémie, l'angine, l'infarctus de myocarde, la maladie chronique du coeur, les insuffisances cardiaques telles que l'insuffisance cardiaque congestive ou de la dégénération maculaire liée à l'âge et l'ostéoporose.

9. Utilisation d'une composition pharmaceutique selon l'une quelconque des revendications 1 à 6 pour le diagnostic des pathologies liées aux phénomènes d'angiogénèse.

10. Utilisation selon la revendication 9 pour le diagnostic des rétinopathies, de l'arthrite rhumatoïde, de la maladie de Crohn, de l'athérosclérose, l'hyperstimulation de l'ovaire, du psoriasis, de l'endométrie associée à la néovascularisation, de la resténose due à l'angioplastie du ballon, de la superproduction tissulaire due à la cicatrisation, de la maladie vasculaire périphérique, de l'hypertension, de l'inflammation vasculaire, de la maladie et les phénomènes de Raynaud, de l'anévrisme, de la resténose artérielle, de la thrombophlébite, de la lymphagyte, du lymphodème, de la cicatrisation et de la réparation tissulaire, de l'ischémie, de l'angine, de l'infarctus de myocarde, de la maladie chronique du cour, des insuffisances cardiaques telles que l'insuffisance cardiaque congestive ou de la dégénération maculaire liée à l'âge et de l'ostéoporose
